# EUROPÄISCHE PATENTSCHRIFT

(11) **EP 1 427 538 B1**
(45) Veröffentlichungstag und Bekanntmachung des Hinweises auf die Patenterteilung: **23.07.2008**
(21) Anmeldenummer: 02767393.8
(22) Anmeldetag: 16.08.2002
(51) Int. Cl.: B05B 11/00, A61M 11/00, A61M 15/00, A61M 5/30

(54) **SPERRSPANNWERK FÜR EINEN MINIATURISIERTEN HOCHDRUCKZERSTÄUBER**
LOCKING-TENSIONING MECHANISM FOR A MINIATURISED HIGH-PRESSURE ATOMISER
MECANISME TENDEUR A BLOCAGE POUR ATOMISEUR HAUTE PRESSION MINIATURISE

(30) Priorität: 04.09.2001 DE 10143350
(43) Veröffentlichungstag der Anmeldung: 16.06.2004
(73) Patentinhaber: Boehringer Ingelheim International GmbH, 55216 Ingelheim am Rhein (DE)
(72) Erfinder: EICHER, Joachim, 44227 Dortmund (DE); FIOL, Andreas, 22850 Norderstedt (DE); SCHYRA, Michael, 42111 Wuppertal (DE); WUTTKE, Gilbert, 44149 Dortmund (DE)
(86) Internationale Anmeldenummer: PCT/EP2002/009146
(87) Internationale Veröffentlichungsnummer: WO 2003/020436

(56) Entgegenhaltungen:
- WO-A-01/64268
- WO-A-82/04409
- WO-A-95/03844
- WO-A-97/12687
- WO-A-97/20590

## Beschreibung

Die Erfindung betrifft ein Sperrspannwerk für eine miniaturisierte Vorrichtung zur Beaufschlagung einer Flüssigkeit mit hohem Druck, wobei das abgegebene Volumen der Flüssigkeit veränderbar ist. Ein derartiger Hochdruckerzeuger kann z.B. in einem Zerstäuber verwendet werden, mit dem ein unter Hochdruck stehendes vorgegebenes Volumen einer Flüssigkeit zu einem Aerosol zerstäubt wird. Weiter kann der Hochdruckerzeuger verwendet werden, um einen Flüssigkeits-Hochdruckstrahl von bevorzugt sehr kleinem Durchmesser zu erzeugen.

Die Flüssigkeit kann zum Beispiel einen pharmazeutischen Wirkstoff enthalten. Die Flüssigkeit kann mit einem Hochdruckzerstäuber zu einem Aerosol zerstäubt werden, z.B. einen pharmazeutischen Aerosol, das über die Lunge oder die Nasenwege aufgenommen wird. Mittels eines nadellosen Injektors kann ein flüssiges Medikament parenteral verabreicht bzw. injiziert werden oder mit der Vorrichtung wird ein Aerosolnebel zur Aufbringung auf ein Auge erzeugt.

Die Erfindung bezweckt, das Einsatzgebiet eines derartigen miniaturisierten Hochdruckerzeugers zu erweitern und die Geräte an Wünsche der Benutzer anzupassen.

Bei den bekannten Sperrspannwerken (W.Krause: Konstruktionselemente der Feinmechanik, Verlag Carl Hanser, München 1993, Seiten 521 bis 523) wird die in einer Feder gespeicherte Energie zum erforderlichen Zeitpunkt freigesetzt und in Bewegung umgesetzt. Die Feder wirkt auf ein geführtes oder gelagertes Bauteil, das als Sprungstück bezeichnet wird. Ein Sperrglied hindert das Sprungstück an der Bewegung und gibt es in vorbestimmter Weise frei.
Aus der WO 97/20590 ist ein Sperrspannwerk für einen federbetätigten Abtrieb bekannt. Dieses Sperrspannwerk umfasst im wesentlichen ein kraftübersetzendes Getriebe, z.B. ein Schraub-Schub-Getriebe, zum Spannen der Feder, mit der die für den vorgegebenen Hochdruck erforderliche Kraft aufgebracht wird, ein ringförmig angeordnetes Sperrglied mit einrückenden Sperrflächen und einer Auslösetaste, ein Sprungstück sowie zwei Anschläge als Wegbegrenzung für das Sprungstück. Diese Bauteile sind in einem zweiteiligen Gehäuse untergebracht; beide Gehäuseteile sind gegeneinander drehbar gelagert. Zum Spannen der Feder werden beide Gehäuseteile (z.B. von Hand) gegeneinander gedreht, wobei durch das Schraub-Schub-Getriebe die Drehbewegung der beiden Gehäuseteile gegeneinander in ein Zusammendrücken der Feder umgewandelt wird. Am Ende der Drehbewegung der beiden Gehäuseteile gegeneinander springt das Sperrglied in seine eingerückte Position und hält das Sprungstück und damit die gespannte Feder in dieser Position. Ist der miniaturisierte Hochdruckerzeuger z.B. ein Zerstäuber gemäß der WO 97/12687, wird die die Aerosolwolke erzeugende Austrittsdüse vor oder in eine Körperhöhle gehalten wird, und der Zerstäubungsvorgang durch Betätigen der Auslösetaste ausgelöst. Ist der Hochdruckerzeuger ein Erzeuger für einen Sprühstrahl z.B. einem nadellosen Injektor gemäß der WO 01/64268, wird die Düse auf ein tierisches oder pflanzliches Gewebe gedrückt, der nadellose Injektor wird durch Betätigen der Auslösetaste ausgelöst, und ein Volumen einer Flüssigkeit wird in das Gewebe injiziert. WO 01/64268 ist Stand der Technik nach Artikel 54(3) EPÜ.

Die Hochdruckerzeuger mit dem in der WO 97/20590 angegebenen Sperrspannwerk geben ein vorgegebenes Volumen einer Flüssigkeit im Mikroliterbereich reproduzierbar ab. Das abgegebene Volumen wird durch die Konstruktion vorgegeben und kann im Nachhinein nicht mehr verändert werden. Eine Veränderung des Volumens ist nur über eine Veränderung der Konstruktion möglich, was mit vergleichsweise großem Aufwand verbunden ist, da die Werkzeuge zum Herstellen der einzelnen Bestandteile an die geänderten Vorgaben anzupassen sind.

Aufgabe der vorliegenden Erfindung ist, einen Hochdruckerzeuger mit Sperrspannwerk mit einer Einstellvorrichtung für das abzugebende Volumen auszustatten, ohne die wesentlichen Bauteile des Sperrspannwerkes oder des Hochdruckerzeugers zu ändern. Das Sperrspannwerk für den federbetätigten Abtrieb umfasst im wesentlichen folgende Bauteile: zwei Gehäuseteile, die gegeneinander bewegbar gelagert sind, eine Arbeitsfeder als Speicher für die mechanische Energie, die auf einen Abtriebsflansch als Sprungstück wirkt, eine Vorrichtung zum Spannen der Arbeitsfeder, einen ersten und einen zweiten Anschlag für das Sprungstück, der die erste beziehungsweise zweite Ruhelage des Sprungstücks bestimmt, einen mit dem Sprungstück verbundenen Kolben, der mit der Bewegung des Sprungstücks in einem Zylinder in axialer Richtung bewegt wird, wobei der sich in Richtung auf eine Austrittsdüse bewegende Kolben das Volumen der Flüssigkeit ausstößt.

Diese Aufgabe wird erfindungsgemäß gelöst durch mindestens ein zusätzliches Bauteil, mit dem die Position einer der beiden Anschläge des Sprungstücks und damit der vom Sprungstück zurückgelegte Weg verändert wird.

Die beiden gegeneinander bewegbaren Gehäuseteile sind um ihre Achse gegeneinander drehbar oder gegeneinander axial verschiebbar gelagert. Die Arbeitsfeder wird bei gegeneinander drehbar gelagerten Gehäuseteilen mittels eines Schraub-Schub-Getriebes gespannt. Bei gegeneinander verschiebbar gelagerten Gehäuseteilen kann das eine Gehäuseteil um ein begrenztes Stück aus dem anderen Gehäuseteil herausgezogen werden, um die Arbeitsfeder zu spannen. Der mit dem Sprungstück verbundene Kolben kann ein massiver Kolben oder ein Hohlkolben sein. Das Sprungstück kann beispielsweise scheibenförmig oder tassenförmig sein.

Der erste Anschlag für das Sprungstück bildet die Wegbegrenzung für das Sprungstück in dessen erster Ruhelage, in der die Arbeitsfeder gespannt ist. Bei diesem Zustand ist das Sperrglied eingerückt. Der zweite Anschlag für das Sprungstück bildet die Wegbegrenzung für das Sprungstück in dessen zweiter Ruhelage, in der die Arbeitsfeder relativ entspannt ist. Bei diesem Zustand ist das Sperrglied ausgerückt. Durch Drehen der beiden Gehäuseteile gegeneinander wird das Sprungstück mittels des Schraub-Schub-Getriebes zum Spannen der Arbeitsfeder von seiner zweiten Ruhelage in seine erste Ruhelage geschoben. Nach dem Ausrücken des Sperrgliedes durch Betätigen der Auslösetaste schiebt die Arbeitsfeder das Sprungstück aus dessen erster Ruhelage in dessen zweite Ruhelage.

Als das mindestens eine zusätzliche Bauteil, mit dem die Lage zum Beispiel des zweiten Anschlages für das Sprungstück verändert wird, kommen in Betracht:
● eine Distanzscheibe oder ein Distanzring mit konstanter Dicke,
● ein Stufenscheibenpaar mit veränderbarer Dicke,
● mehrere außerhalb der Geräteachse im Boden des tassenförmigen Sprungstücks oder im Gehäuseteil, welches das Sperrglied enthält, angeordnete Stifte oder Schrauben,
● eine Stellmutter, die auf das Sprungstück geschraubt wird,
● eine verstellbare Anschlagplatte,
● eine zentrisch zur Geräteachse angeordnete Schraube,
● ein mehrstufig veränderbarer zweiter Anschlag, mit dem das von dem Hochdruckerzeuger abzugebende Volumen in mehrere Teile aufgeteilt werden kann,
● ein Schraub-Schub-Getriebe zum Einstellen eines Anschlags des Sprungstücks, wobei das Schraub-Schub-Getriebe einen drehbaren Stellring und einen axial verschiebbaren Ring umfasst, die beide jeweils eine Schraubenfläche haben.

Das Schraub-Schub-Getriebe zum Einstellen eines Anschlags des Sprungstücks ist zu unterscheiden von dem oben beispielhaft erwähnten Schraub-Schub-Getriebe zum Spannen der Arbeitsfeder.

Beispielsweise kann die zweite Ruhelage des Sprungstücks auf folgende Weise verändert werden:
Auf den Boden des tassenförmigen Sprungstücks, bevorzugt in Form eines nach einer Seite hin offnen Zylinders, wird eine Scheibe oder ein Ring mit konstanter Dicke gelegt. Der Weg des Sprungstücks wird um die Dicke der Scheibe oder des Ringes verändert. Die Scheibe oder der Ring kann in das Sprungstück eingeklemmt oder eingeklebt sein. Der Rand der Scheibe oder des Ringes kann glatt oder mit Klemmzacken versehen sein.

Das Sprungstück wird in seinem Innenboden mit mehreren Sacklöchern versehen (z.B. mit drei Löchern, die azimutal um jeweils 120 Grad gegeneinander versetzt sind). In die Sacklöcher werden Stifte eingesetzt, die aus den Sacklöchern hervorstehen. Der Weg des Sprungstücks wird um den aus den Sacklöchern hervorstehenden Teil der Stifte geändert.
In die Sacklöcher werden Schrauben eingedreht. Der Weg des Sprungstücks wird um den aus den Sacklöchern hervorstehenden Teil der Schrauben verändert.
Der Boden des Sprungstücks wird mit mehreren (z.B. drei) durchgehenden Löchern versehen, in die von der der Feder zugewandten Seite des Sprungstücks Schrauben gedreht werden, die in das Innere des Sprungstücks hineinragen. Der Weg des Sprungstücks wird um die Länge der in das Innere des Sprungstücks ragenden Abschnitte der Schrauben verändert.

Vom düsenseitigen Ende des Hochdruckerzeugers werden Schrauben in das obere Gehäuseteil gedreht, die auf den zweiten Anschlag für das Sprungstück wirken. Diese Schrauben können von außen zugänglich sein.

Eine zentrische Schraube mit darin befestigtem Hohlkolben wird in den Boden des Sprungstücks gedreht. Der in das Innere des tassenförmigen Sprungstücks ragende Teil der zentrischen Schraube verändert den Weg des Sprungstücks. Bei dieser Ausführungsform der Erfindung wird der Hohlkolben zwangsläufig um dasselbe Stück weiter in den Zylinder hineingeschoben. Das Totvolumen vor dem Ende des Hohlkolbens, das der Austrittsdüse zugewandt ist, bleibt praktisch unverändert.

Die Position eines der beiden Anschläge des Sprungstücks des Sperrspannwerks kann weiter mittels eines drehbaren Stellrings, der mit einem axial verschiebbaren Ring nach Art eines Schraub-Schub-Getriebes zusammenwirkt, verändert werden. Der drehbare Stellring hat einen Griff, der aus dem Gehäuse herausragt, und der von außen zugänglich ist. Der drehbare Stellring und der axial verschiebbare Ring haben jeweils eine Schraubenfläche, in der beide Ringe gegeneinander gleiten. Die Schraubenfläche kann eine Rechtsschraube oder eine Linksschraube sein.
Der drehbare Stellring ist innerhalb des Gehäuses drehbar gelagert. Er kann an seiner der Schraubenfläche gegenüber liegenden Seite axial gegen das Gehäuse abgestützt sein. Der axial verschiebbare Ring ist innerhalb des Gehäuses gegen Drehen gesichert. Die der Schraubenfläche gegenüber liegende Seite des verschiebbaren Rings ist einer der beiden Anschläge des Sprungstücks des Sperrspannwerks, und zwar entweder der erste oder der zweite Anschlag.
Der drehbare Stellring kann innerhalb eines vorgegebenen Winkelbereiches in beiden Drehrichtungen beliebig eingestellt werden. Der vorgegebene Drehbereich ist konstruktionsbedingt begrenzt, zum Beispiel auf weniger als 180 Grad, bevorzugt auf weniger als 90 Grad.
Beim Drehen des drehbaren Stellrings wird der Abstand der Anschlagfläche des axial verschiebbaren Rings von der Seite, an der sich der drehbare Stellring gegen das Gehäuse abstützt, verändert. Damit wird die Position des ersten oder des zweiten Anschlags des Sprungstücks des Sperrspannwerks reproduzierbar verändert und der vom Sprungstück zurücklegbare Weg eingestellt.
Die Schraubenfläche des Schraub-Schub-Getriebes kann eine eingängige Schraubenfläche sein, wenn der Drehwinkel des drehbaren Stellrings kleiner als zum Beispiel 90 Grad ist. Aus Gründen der Festigkeit und der gleichmäßigen Verteilung der Kraft innerhalb des Schraub-Schub-Getriebes kann es zweckmäßig sein, die Schraubenfläche als mehrgängige Schraubenfläche auszubilden, zum Beispiel als zweigängige, dreigängige oder viergängige Schraubenfläche.
Die Schraubenflächen am drehbaren Stellring und am axial verschiebbaren Ring können durchgehend vorhanden sein, bei einer eingängigen Schraubenfläche über einen Winkel bis zu 360 Grad, bei einer zweigängigen Schraubenfläche bis zu einem Winkel von jeweils 180 Grad, und bei einer viergängigen Schraubenfläche bis zu einem Winkel von jeweils 90 Grad. Weiter können die Schraubenflächen an einem oder beiden Ringen nur stückweise über einen kleineren dieser Winkel vorhanden sein.
Die Ganghöhe der Schraubenflächen ist bei einem Paar zusammenwirkender Ringe gleich groß. Die Ganghöhe kann innerhalb eines konstruktionsbedingten Bereiches frei gewählt werden. Innerhalb konstruktionsbedingter Grenzen kann der Weg, den der axial verschiebbare Ring bei einem vorgegebenen Drehwinkel des drehbaren Rings zurücklegt, über eine angepasste Ganghöhe der Schraubenfläche eingestellt werden.
Das Schraub-Schub-Getriebe kann eine Rastung - bevorzugt zum Beispiel in Form von Rastzähnen - haben, die das unbeabsichtigte Verstellen des Schraub-Schub-Getriebes verhindert. Ein derartiges Getriebe ist erst verstellbar, wenn am drehbaren Stellring eine hinreichend große Drehkraft angreift. Die Rastung kann an der Seite des drehbaren Stellrings angebracht sein, an der sich der Stellring am Gehäuse abstützt. Weiter kann die Rastung an den Schraubenflächen - bevorzugt zum Beispiel in Form von Rastzähnen oder Raststufen - angebracht sein.
Falls die Schraubenflächen jeweils auf einer Kante jedes Rings angebracht sind, kann der drehbare Stellring den axial verschiebbaren Ring nur vor sich herschieben, der drehbare Stellring kann den axial verschiebbaren Ring nicht zurückziehen. Bei dieser Ausführung des Schraub-Schub-Getriebes kann der axial verschiebbare Ring mittels einer Rückstellfeder an den Stellring gedrückt werden.

In einer anderen Ausführung können die Schraubenflächen in oder auf der zylindrischen Wand der Ringe angebracht sein, zum Beispiel als zweigängige Kulissen in dem einen Ring und als zweigängige Nocken in dem anderen Ring. Die Nocken gleiten in den Kulissen, und der axial verschiebbare Ring kann beim Drehen des drehbaren Stellrings in beiden axialen Richtungen verschoben werden. Bei dieser Ausführung ist keine Rückstellfeder erforderlich. Die Nocken können zum Beispiel schraubenflächenartig geformt sein; weiter können sie als aus der Wand hervorstehende Zylinder geformt sein.

Bei einer weiteren Ausführung ist es möglich, die eine Schraubenfläche nicht auf dem axial verschiebbaren Ring anzubringen, sondern direkt auf der Seite des Sprungstücks, das dem drehbaren Stellring zugewandt ist, sofern das axial verschiebbare Sprungstück innerhalb des Gehäuses gegen Drehen gesichert ist. Bei dieser Ausführung kann entweder der erste Anschlag oder der zweite Anschlag des Sprungstücks als Schraubenfläche ausgeführt werden, und folglich kann die Position des ersten Anschlags oder des zweiten Anschlags verändert werden.

Das erfindungsgemäße Sperrspannwerk hat folgende Vorteile:
● Je nach Ausführungsform der Erfindung kann das von dem Hochdruckerzeuger abzugebende Volumen während der Montage des Hochdruckerzeugers, oder vor dessen Benutzung oder während dessen Benutzung auf einfache Weise verändert werden.
● Das abzugebende Volumen der Flüssigkeit kann entweder kontinuierlich oder stufenweise verändert werden.
● Der Arbeitsbereich der Arbeitsfeder beim Ausstoßen des Volumens der Flüssigkeit beginnt bei der erfindungsgemäßen Veränderung der zweiten Ruhelage des Sprungstücks bei der großen Federspannung. Die Teilchengrößenverteilung des von dem Hochdruckzerstäuber erzeugten Aerosols oder die Geschwindigkeit, die der nadellose Injektor dem ausgestoßenen Volumen der Flüssigkeit erteilt, bleiben durch die Änderung des zweiten Anschlages für das Sprungstück praktisch unverändert.
● Bei der erfindungsgemäßen Änderung der Lage des zweiten Anschlages für das Sprungstück bleiben die Lage des ersten Anschlages für das Sprungstück sowie Art und Wirkungsweise des Sperrglieds unverändert.
● Wenn bei einem Sperrspannwerk für einen Hochdruck-Zerstäuber oder für einen nadellosen Injektor nicht nur der zweite Anschlag für das Sprungstück geändert wird, sondern im gleichen Ausmaß die Position des (Hohl)-Kolbens in Bezug auf das Sprungstück, kann das Totvolumen für die Flüssigkeit, die sich innerhalb des Zylinders zwischen dem der Austrittsdüse zugewandten Ende des (Hohl)-Kolbens und dem Düsenkörper befindet, auf einem vorgegebenen Wert gehalten werden.

Die Erfindung wird bevorzugt in einem Hochdruckzerstäuber gemäß der Patentanmeldung WO 91/14468 oder der WO 97/12687, d.h. Zerstäuber zur Erzeugung von pharmazeutischen Aerosolen zur inhalativen oder nasalen Aufnahme, einem nadellosen Injektor gemäß der WO 01/64268 oder einer Augensprayvorrichtung gemäß der PCT/EP0207038 eingesetzt.

Den genannten Geräten liegt der gleiche Mechanismus zur Beaufschlagung von großen Drücken auf eine vorgegebene Menge einer Flüssigkeit zugrunde. Beispielhaft soll deshalb an dieser Stelle der genannte Hochdruckzerstäuber besprochen werden. Im Fall von unterschiedlichen oder nicht miteinander vereinbarbaren Konstruktionsbeschreibungen zwischen dem vorstehend oder in den Figuren beschriebenen Ausführungsformen der Erfindung und den nun folgenden Ausführungsformen, sind die vorstehend genannten Ausführungsformen und die-in den Figuren genannten Ausführungsformen gegenüber den nun folgenden Ausführungsformen bevorzugt.

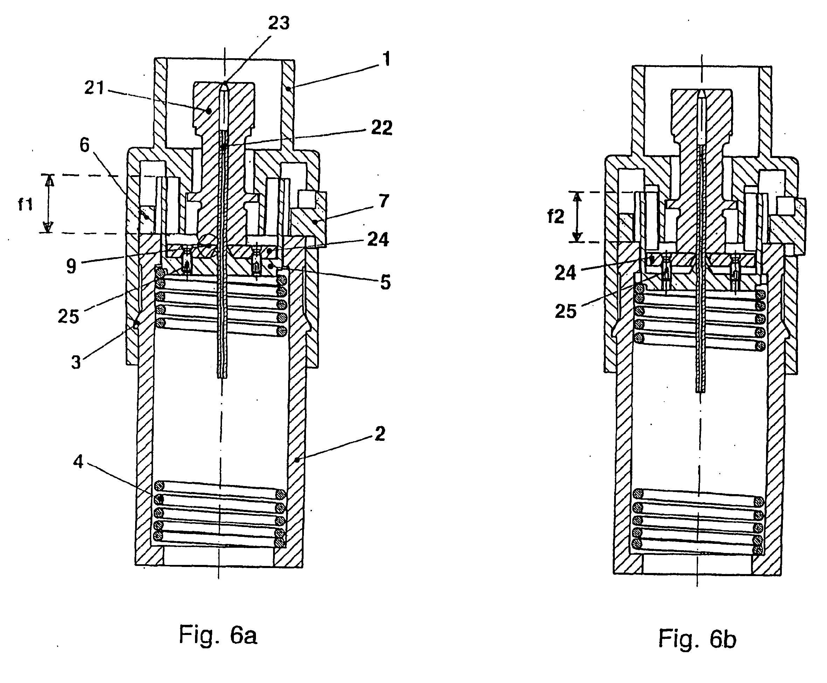

Eine derartige Vorrichtung zur treibgasfreien Vernebelung einer dosierten Menge eines flüssigen Arzneimittels, wird beispielsweise in der internationalen Patentanmeldung WO 91/14468 "Atomizing Device and Methods" als auch in der WO 97/12687, dort Figuren 6a und 6b und den dazugehörigen Beschreibungen, ausführlich beschrieben. In einem solchen Vernebler wird eine Arzneimittellösung mittels hohen Drucks von bis zu 50 MPa (500 bar) in ein Aerosol mit einer mittleren Teilchengrößen (mittlerer aerodynamische Durchmesser) von weniger als 20 Mikrometern überführt und versprüht. Auf die genannten Referenzen wird im Rahmen der vorliegenden Erfindungsbeschreibung ausdrücklich in Gänze Bezug genommen.

In solchen Verneblern werden die Lösungsformulierungen in einem Reservoir gelagert. Dabei ist es notwendig, dass die verwendeten Wirkstoffformulierungen eine ausreichende Lagerstabilität aufweisen und gleichzeitig so beschaffen sind, dass sie dem medizinischen Zweck entsprechend möglichst ohne weitere Manipulation, direkt appliziert werden können. Ferner dürfen sie keine Bestandteile aufweisen, die so mit dem Vernebler wechselwirken können, dass der Vernebler oder die pharmazeutische Qualität der Lösung, respektive des erzeugten Aerosols, Schaden nehmen könnte.

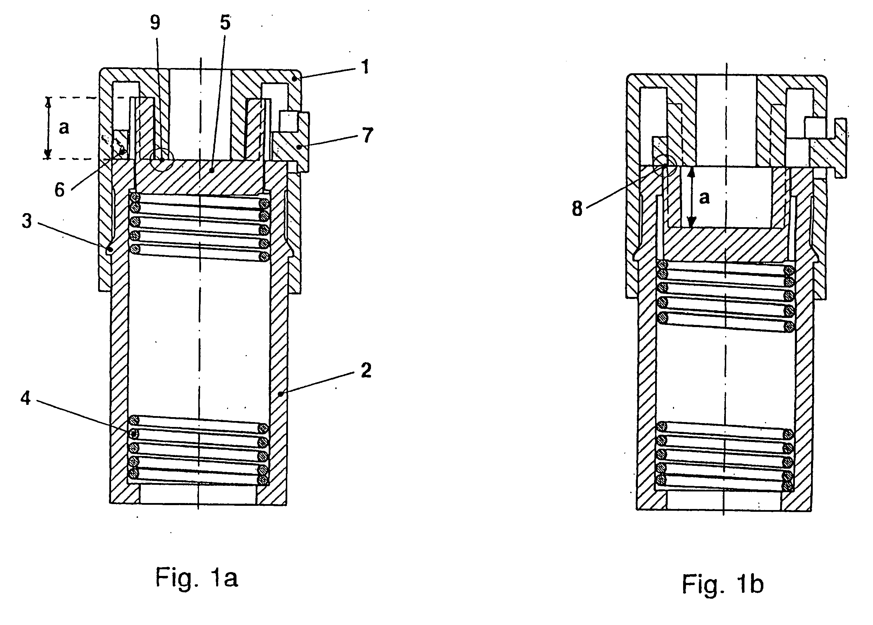

Zur Vernebelung der Lösung wird eine spezielle Düse verwendet, wie sie beispielsweise die WO 94/07607 oder die WO 99/16530, dort besonders Figur 1 und die dazugehörende Beschreibung, beschreiben, auf die beide hiermit ausdrücklich Bezug genommen wird.

Im wesentlichen besteht der bevorzugte Zerstäuber aus einem Gehäuseoberteil, einem Pumpengehäuse, einer Düse, einem Adapter, dem erfindungsgemäßen Sperrspannwerk, einem Federgehäuse, einer Feder und einem Vorratsbehälter, wobei die hervorstechenden Merkmale des Verneblers sind:
- ein Pumpengehäuse, das im Gehäuseoberteil befestigt ist und das an seinem einen Ende einen Düsenkörper mit der Düse bzw. Düsenanordnung trägt,
- einen Hohlkolben mit Ventilkörper,
- einen Abtriebsflansch, in dem der Hohlkolben befestigt ist, und der sich im Gehäuseoberteil befindet,
- dem erfindungsgemäßen Sperrspannwerk, das sich im Gehäuseoberteil befindet,
- ein Federgehäuse mit der darin befindlichen Feder, das am Gehäuseoberteil mittels eines Drehlagers drehbar gelagert ist,
- ein Gehäuseunterteil, das auf das Federgehäuse in axialer Richtung aufgesteckt ist und
- einem Adapter in Form einen Hohlraums mit zwei gegenüberliegenden Öffnungen, wobei die kleinere Öffnung wenigstens die Austrittstelle des Aerosols aus der Düse schlüssig umgibt und die größere Öffnung eine Kontur aufweist, die es ermöglicht diese Öffnung über ein Auge zu stülpen.

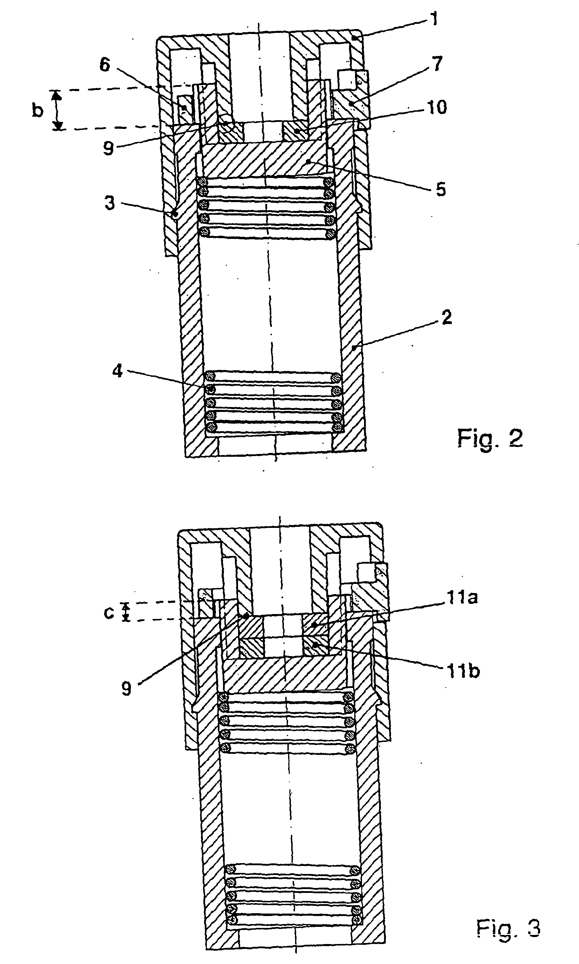

Der Hohlkolben mit Ventilkörper entspricht einer in der WO 97/12687 offenbarten Vorrichtungen. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 MPa (etwa 50 bis 600 bar), bevorzugt 10 bis 60 MPa (etwa 100 bis 600 bar) auf das Fluid, die abgemessene Wirkstofflösung aus. Dabei werden Volumina von 10 bis 50 Mikroliter bevorzugt, besonders bevorzugt sind Volumina von 5 bis 20 Mikroliter, ganz besonders bevorzugt ist ein Volumen von 15 Mikroliter pro Hub.

Der Ventilkörper ist bevorzugt an dem Ende des Hohlkolbens angebracht, das dem Düsenkörper zugewandt ist.

Die Düse im Düsenkörper ist bevorzugt mikrostrukturiert, d.h. durch Mikrotechnik hergestellt. Mikrostrukturierte Düsenkörper sind beispielsweise in der WO 94/07607 offenbart; auf diese Schrift wird hiermit inhaltlich Bezug genommen, insbesondere auf Figur 1 und deren Beschreibung.

Der Düsenkörper besteht z.B. aus zwei fest miteinander verbundenen Platten aus Glas und/oder Silizium, von denen wenigstens eine Platte einen oder mehrere mikrostrukturierte Kanäle aufweist, die die Düseneinlassseite mit der Düsenauslassseite verbinden. Auf der Düsenauslassseite ist mindestens eine runde oder nicht-runde Öffnung von 2 bis 10 Mikrometer Tiefe und 5 bis 15 Mikrometern Breite, wobei die Tiefe bevorzugt bei 4, 5 bis 6,5 Mikrometern und die Breite bei 7 bis 9 Mikrometern beträgt.
Im Fall von mehreren Düsenöffnungen, bevorzugt sind zwei, können die Strahlrichtungen der Düsen im Düsenkörper parallel zueinander verlaufen oder sie sind in Richtung Düsenöffnung gegeneinander geneigt. Bei einem Düsenkörper mit mindestens zwei Düsenöffnungen auf der Auslassseite können die Strahlrichtungen mit einem Winkel von 20 Grad bis 160 Grad gegeneinander geneigt sein, bevorzugt wird ein Winkel von 60 bis 150 Grad, insbesondere bevorzugt 80 bis 100°.
Die Düsenöffnungen sind bevorzugt in einer Entfernung von 10 bis 200 Mikrometern angeordnet, stärker bevorzugt in einer Entfernung von 10 bis 100 Mikrometer, besonders bevorzugt 20 bis 50 Mikrometer. Am stärksten bevorzugt sind 22 bis 28 Mikrometer.
Die Strahlen treffen einander dementsprechend unmittelbar vor der Düsenöffnungen.

Die flüssige Arzneimittelzubereitung steht wie bereits erwähnt unter einem Eingangsdruck von bis zu 60 MPa (600 bar) bevorzugt 20 bis 30 MPa (200 bis 300 bar) am Eingang des Düsenkörpers und wird über die Düsenöffnungen in ein inhalierbares Aerosol zerstäubt. Die bevorzugten Teilchengrößen des Aerosols liegen bei bis zu 20 Mikrometern, bevorzugt 3 bis 10 Mikrometern.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseoberteil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganz zahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere das zu zerstäubende Fluid enthaltende austauschbare Vorratsbehälter eingeschoben und benutzt werden. Der Vorratsbehälter enthält die erfindungsgemäße wässerige Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Kolben in den Zylinder des Pumpengehäuses hinein. Das Fluid tritt aus der Düse des Zerstäubers in zerstäubter Form aus.

Gegebenenfalls kann ein Teil der Elemente des Verneblers, die mit der zu applizierenden Flüssigkeit auf ihrem Weg vom Vorratsbehälter zur Düse in Kontakt kommen, aus oligodynamisch aktiven Bestandteilen gefertigt werden oder mit keimtötenden Materialien beschichtet sein. Alternativ dazu oder in Ergänzung kann in diesen Weg ein keimabweisender Filter ausgebildet sein. Solche Ausführungsformen haben den Vorteil, dass keine Keime von Außen in das Vorrastgefäß eindringen können und dadurch auf den Zusatz von Konservierungsmitteln verzichtet werden kann. Dies ist insbesondere für Langzeitanwendungen vorteilhaft, wie eingangs geschildert.

Weitere konstruktive Details sind in den PCT-Anmeldungen WO 97/12687 und WO 97/20590 offenbart, auf die hiermit noch einmal inhaltlich Bezug genommen wird.

Die Bauteile des Zerstäubers (Verneblers) sind aus einem der Funktion entsprechend geeignetem Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

### Figuren

Die Erfindung wird an Hand der Figuren weiter erläutert. Figur 1a und Figur 1b zeigen einen Längsschnitt durch ein Sperrspannwerk nach dem Stand der Technik, also ohne ein weiteres Bauteil gemäß der Erfindung. Das obere zylindrische Gehäuseteil (1) greift über das Federgehäuse (2), mit dem es über Schnappnasen (3) verbunden ist. Die Schnappnasen (3) sind auf der Außenseite des Federgehäuses (2) angebracht und können sich über zwei einander gegenüber liegende Kreissegmente von jeweils etwa 30 Grad erstrecken. Sie greifen in eine umlaufende Rille auf der Innenseite des oberen Gehäuseteils (1) ein. Die beiden Gehäuseteile sind also gegeneinander drehbar. Im Federgehäuse befindet sich die Druckfeder (4), die im allgemeinen bereits beim Zusammenstecken des beiden Gehäuseteile vorgespannt wird. Die Druckfeder (4) stützt sich auf einem umlaufenden Vorsprung am unteren Ende des Federgehäuses ab sowie auf dem Sprungstück (5), das zwischen dem oberen Gehäuseteil und dem Federgehäuse achsenparallel verschiebbar angeordnet ist, und das seinerseits gegen das obere Gehäuseteil (1) drückt. Das tassenförmige Sprungstück (5) ragt in das obere Gehäuseteil (1) hinein. Das ringförmige Sperrglied (6) umschließt das Sprungstück. Die am Sperrglied angebrachte Auslösetaste (7) ragt aus dem oberen Gehäuseteil seitlich hervor. Der Kragen des tassenförmigen Sprungstücks ist z.B. auf seiner Innenseite mit beispielsweise zwei sägezahnförmigen Aussparungen versehen, auf denen zwei Sägezähne im oberen Gehäuseteil abgleiten. Die Sägezähne und die Aussparungen sind in Figur 1a stark vereinfacht dargestellt. Beim Drehen des oberen Gehäuseteils gegen das Federgehäuse wird das axial verschiebbare Sprungstück gegen die Kraft der Druckfeder weiter in das Federgehäuse hineingedrückt. Sobald der obere Rand des tassenförmigen Sprungstücks durch das Sperrglied hindurch weit genug nach unten gedrückt worden ist, schiebt sich das ringförmige Sperrglied senkrecht zur Gehäuseachse (gestrichelte Linie in den Figuren) zwischen den oberen Rand des tassenförmigen Sprungstücks und einen ringförmigen Vorsprung im oberen Gehäuseteil und hält das Sprungstück und die durch die Verschiebung des Sprungstücks (zusätzlich) gespannte Druckfeder in der erreichten Position fest.
Durch Drücken der Auslösetaste (7) wird das ringförmige Sperrglied (6) senkrecht zur Gehäuseachse zurückgeschoben, wodurch der Weg des Sprungstücks freigegeben wird.

Die Druckfeder schiebt das Sprungstück über eine vorgegebene Strecke nach oben durch das ringförmige Sperrglied hindurch und betätigt damit ein in Figur 1 a und Figur 1b nicht dargestelltes mit dem Sprungstück verbundenes Bauteil, z.B. verschiebt es einen Kolben in einem Zylinder.

In Figur 1 a ist das Sperrspannwerk mit dem Sprungstück in seiner zweiten Ruhelage und mit ausgerücktem Sperrglied dargestellt. Figur 1b zeigt das Sperrspannwerk mit dem Sprungstück in seiner ersten Ruhelage und eingerücktem Sperrglied. Der erste Anschlag (8) ist die Wegbegrenzung für das Sprungstück in dessen erster Ruhelage, der zweite Anschlag (9) ist die Wegbegrenzung für das Sprungstück in dessen zweiter Ruhelage. Durch Drehen der beiden Gehäuseteile gegeneinander geht der Zustand nach Figur 1a in den Zustand nach Figur 1b über. Nach Drücken der Auslösetaste geht durch die Kraft der gespannten Druckfeder der Zustand nach Figur 1b in den Zustand nach Figur 1a über. In den Figuren 1a und 1b ist die Strecke (a) der vom Sprungstück zurückgelegte Weg.

Die Figuren 2 bis 7 zeigen verschiedene erfindungsgemäße Sperrspannwerke, bei denen der Weg des Sprungstücks veränderbar ist.
Figur 2 zeigt das Sprungstück (5) in seiner zweiten Ruhelage, das Sprungstück liegt am zweiten Anschlag (9) an. Zusätzlich zu den beschriebenen Bauteilen ist der flache Ring (10) auf dem Boden des tassenförmigen Sprungstücks (5) vorhanden. Dieser Ring wird während der Montage des Sperrspannwerks in das Sprungstück eingelegt. Der Weg (b) des Sprungstücks ist um die Dicke des flachen Ringes (10) verändert. Durch den Ring (10) wird die Menge einer Flüssigkeit, die ein mit dem Sprungstück (5) verbundener (nicht dargestellter) Kolben ausstoßen kann, verändert.
Figur 3 zeigt ebenfalls das Sprungstück (5) in seiner zweiten Ruhelage, das Sprungstück liegt am zweiten Anschlag (9) an. Auf dem Boden des tassenförmigen Sprungstücks liegt eine zweiteilige Stufenscheibe (11a; 11b), die während der Montage des Sperrspannwerks in das Sprungstück gelegt wird. Jede der beiden Scheiben (11a) und (11b) enthält auf den einander zugewandten Seiten mehrere Stufen, z.B. vier Stufen, von denen jede Stufe auf jeder Scheibe mehrfach, z.B. dreifach, vorhanden ist. Die jeweils vier Stufen gleicher Höhe sind um einen Winkel von jeweils 120 Grad gegeneinander versetzt. Liegt die höchste Stufe der einen Scheibe auf der niedrigsten Stufe der anderen Scheibe, hat die zweiteilige Stufenscheibe ihre kleinste Dicke. Liegt die höchste Stufe der einen Scheibe auf der höchsten Stufe der anderen Scheibe, hat die zweiteilige Stufenscheibe ihre größte Dicke. Der Weg (c) des Sprungstücks ist um die Dicke der zweiteiligen Stufenscheibe (11a; 11b) verändert.

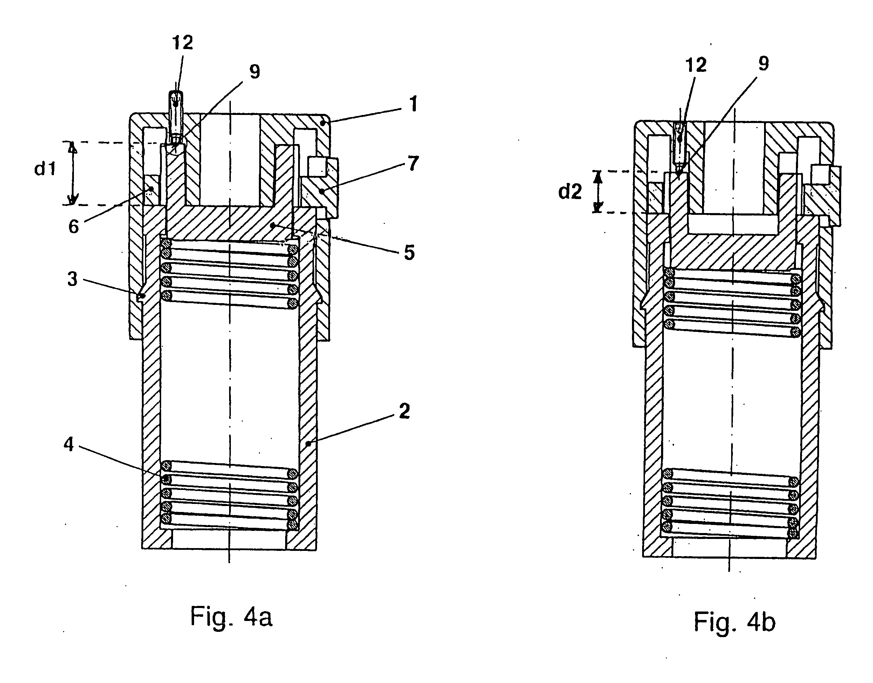

Die Figuren 4a und 4b zeigen das Sprungstück (5) in seiner zweiten Ruhelage, das Sprungstück liegt am zweiten Anschlag (9) an. In das obere Gehäuseteil (1) sind mehrere Stellschrauben (12) gedreht, die z.B. bei drei Schrauben jeweils 120 Grad gegeneinander versetzt sind. In den Figuren 4a und 4b ist nur jeweils eine dieser Schrauben dargestellt. Die Stellschrauben (12) sind auch nach der Montage des Sperrspannwerks von außen zugänglich. Alle Stellschrauben (12) an einem Sperrspannwerk werden gleich tief eingedreht, jedoch kann die Eindrehtiefe innerhalb des Stellbereiches unterschiedlich vorgegeben und kontinuierlich verändert werden. Die innerhalb des Gehäuseoberteils (1) befindlichen Enden der Stellschrauben bestimmen den zweiten Anschlag (9) für das Sprungstück. In Figur 4a sind die Stellschrauben (12) weniger tief eingedreht als in Figur 4b. Der Weg (d1) des Sprungstücks in Figur 4a ist länger als der Weg (d2) des Sprungstücks in Figur 4b.

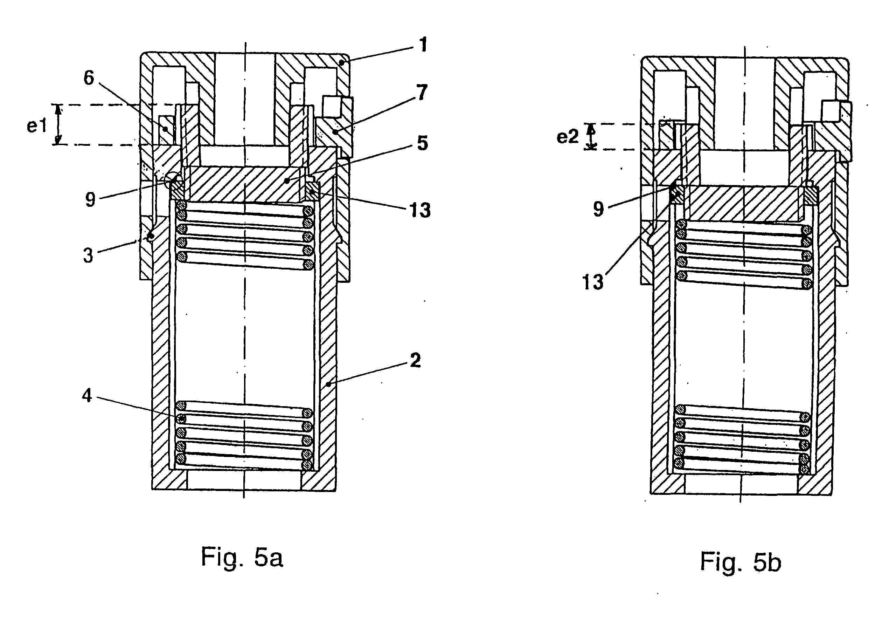

Die Figuren 5a und 5b zeigen das Sprungstück (5) in seiner zweiten Ruhelage. Das Sprungstück ist an seinem der Feder (4) zugewandten Ende mit einer Stellmutter (13) versehen, die unterschiedlich weit auf das Sprungstück gedreht werden kann. In der zweiten Ruhelage des Sprungstücks liegt die obere Seite der Stellmutter (13) am Anschlag (9) an. Je weiter die Stellmutter (13) auf das Sprungstück geschraubt wird, desto kürzer wird der Weg des Sprungstücks. Der Weg (e2) ist kürzer als der Weg (e1). Die Figuren 6a und 6b zeigen außer dem Sperrspannwerk einen Zylinder (21), der im oberen Gehäuseteil (1) befestigt ist, und einen Hohlkolben (22), der im Sprungstück (5) befestigt ist. Am Ende des Zylinders befindet sich die Düse (23). Der Weg des Hohlkolbens ist also genau so lang wie der Weg des Sprungstücks. Auf dem Boden des tassenförmigen Sprungstücks liegt eine Platte (24), deren Abstand vom Boden des tassenförmigen Sprungstücks mittels Stellschrauben (25) kontinuierlich veränderbar ist. Die Stellschrauben (25) sind nach der Montage des Sperrspannwerks von außen zugänglich. Je weiter die Platte (24) vom Boden des tassenförmigen Sprungstücks entfernt liegt, desto kürzer ist der Weg des Sprungstücks. Der Weg (f2) ist kürzer als der Weg (f1). Die Figuren 6a und 6b stehen in einem inhaltlichen Zusammenhang mit den Figuren 6a und 6b der WO 97/12687, die einen Vernebler darstellen, der bevorzugt als Inhalator eingesetzt wird. Auf diese Zeichnungen und die dazugehörigen Ausführungen wird im Rahmen der vorliegenden Erfindungen ausdrücklich Bezug genommen. Gleichfalls Bezug genommen wird auf die Figuren 1 und 2 der WO 01/64268, die einen nadellosen Injektor darstellen und auf die Figuren 4 und 5 der PCT/EP0207038, die eine Vorrichtung zur Erzeugung eines Augensprays beschreiben.

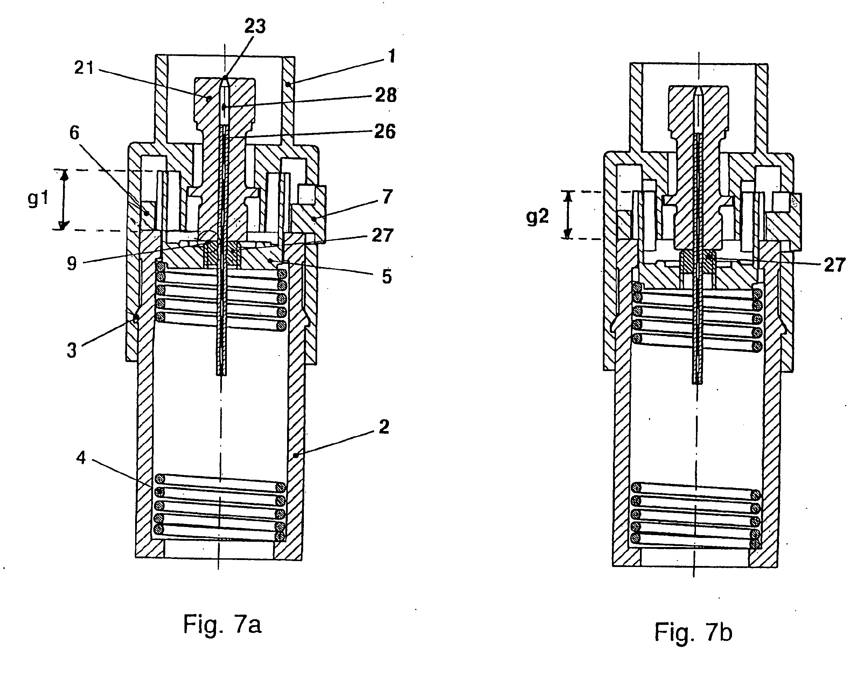

Die Figuren 7a und 7b zeigen ein Sperrspannwerk, einen im Gehäuseoberteil (1) befestigten Zylinder (21) sowie einen Hohlkolben (26). Der Hohlkolben ist in der zentrischen Stellschraube (27) befestigt, die unterschiedlich tief in den Boden des tassenförmigen Sprungstücks geschraubt werden kann. Die zentrische Stellschraube (27) ist nach der Montage des Sperrspannwerks von außen zugänglich. Je tiefer die zentrische Stellschraube in den Boden des tassenförmigen Sprungstücks geschraubt wird, desto kürzer ist der Weg des Sprungstücks und damit der Weg des Hohlkolbens innerhalb des Zylinders. Bei dieser Anordnung des Hohlkolbens bleibt das Totvolumen (28) zwischen Düse und dem düsenseitigen Ende des Hohlkolbens unverändert, wenn die zentrische Stellschraube unterschiedlich tief in den Boden des Sprungstücks gedreht wird. Der Weg (g2) ist kürzer als der Weg (g1).

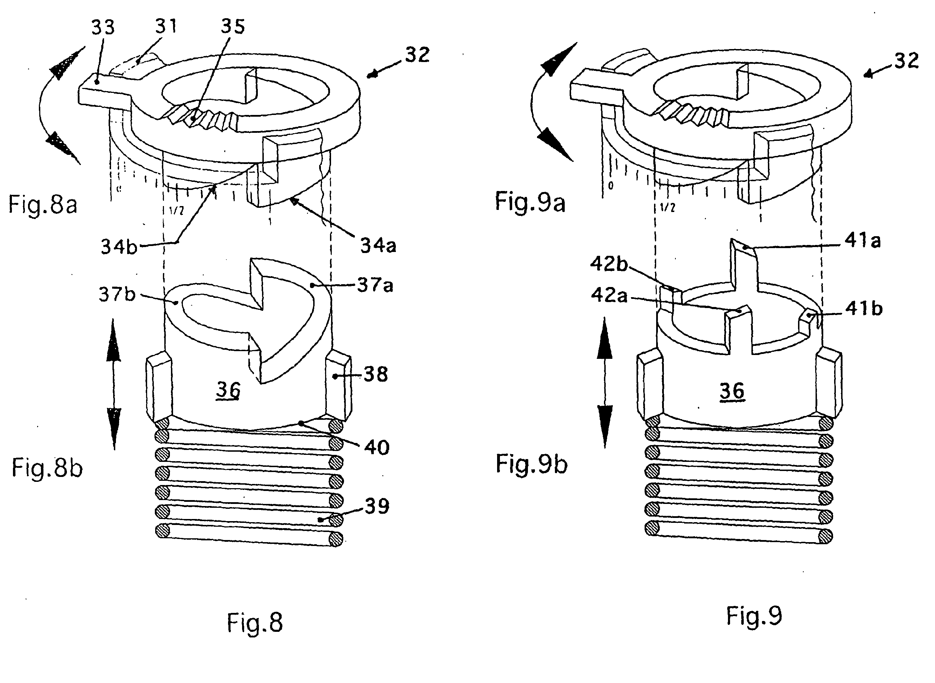

In den Figuren 8 bis 14 sind Einzelheiten eines Schraub-Schub-Getriebes zum Einstellen eines Anschlages des Sprungstücks dargestellt.
Figur 8 zeigt den drehbaren Stellring (32) und den axial verschiebbaren Ring (36) in Sprengdarstellung. Der in Figur 8a gezeigte drehbare Stellring (32) befindet sich innerhalb des (nur teilweise dargestellten) Gehäuses (31). Der Griff (33) des drehbaren Stellrings ist von außen zugänglich und kann innerhalb eines Schlitzes im Gehäuse verschoben werden. Auf der einen Kante des drehbaren Stellrings sind Rastzähne (35) vorhanden, die mit (nicht dargestellten) Rastzähnen im Gehäuse zusammenwirken. Die andere Kante des drehbaren Stellrings ist eine zweigängige Schraubenfläche (34a; 34b). Jeder der beiden Schraubengänge erstreckt sich über einen Winkel von 180 Grad.

Der in Figur 8b gezeigte verschiebbare Ring (36) hat auf der Kante, die dem drehbaren Stellring (32) zugewandt ist, ebenfalls eine zweigängige Schraubenfläche (37a; 37b). Jeder der beiden Schraubengänge erstreckt sich über einen Winkel von 180 Grad. Die zweigängige Schraubenfläche des axial verschiebbaren Rings hat dieselbe Ganghöhe wie die zweigängige Schraubenfläche des drehbaren Stellrings.
Der axial verschiebbare Ring ist mit Führungsstegen (38) gegen Drehen gesichert. Die Führungsstege gleiten in (nicht dargestellten) Nuten im Gehäuse.
Die Schraubenfläche des axial verschiebbaren Rings (36) wird durch die Rückstellfeder (39) gegen die Schraubenfläche des drehbaren Stellrings gedrückt. Die Schraubenfeder ist im Querschnitt dargestellt. Beim Drehen des drehbaren Stellrings gleitet die Schraubenfläche (34a) auf der Schraubenfläche (37a) und die Schraubenfläche (34b) auf der Schraubenfläche (37b).
Die ebene Kante (40) des axial verschiebbaren Ringes (36) ist der Anschlag für das (nicht dargestellte) Sprungstück des Sperrspannwerks.

In Figur 9 ist eine weitere Ausführung eines Schraub-Schub-Getriebes zum Einstellen eines Anschlages des Sprungstücks dargestellt. Die Figur 9a stimmt mit der Figur 8a überein. In Figur 9b sind von der Schraubenfläche (37a; 37b) in Figur 8b nur die Endflächen (41a; 41b) und (42a; 42b) an den vier Vorsprüngen erhalten geblieben. Die restlichen Bereiche der Schraubenfläche (37a; 37b) sind weggeschnitten. Die (im Querschnitt dargestellte) Schraubenfeder (40) drückt die Endflächen (41a; 41b) gegen die Schraubenfläche (34a) und die Endflächen (42a; 42b) gegen die Schraubenfläche (34b) des drehbaren Stellrings.
Die azimutalen Abstände zwischen den beispielsweise vier Vorsprüngen können jeweils 90 Grad betragen. Gegebenenfalls können die azimutalen Abstände unterschiedlich groß sein. Die freien Räume zwischen den Vorsprüngen können für andere Zwecke benutzt werden, zum Beispiel für Bauelemente, die zwischen der Gehäusewand und dem inneren Bereich der beiden Ringe vorgesehen sind.

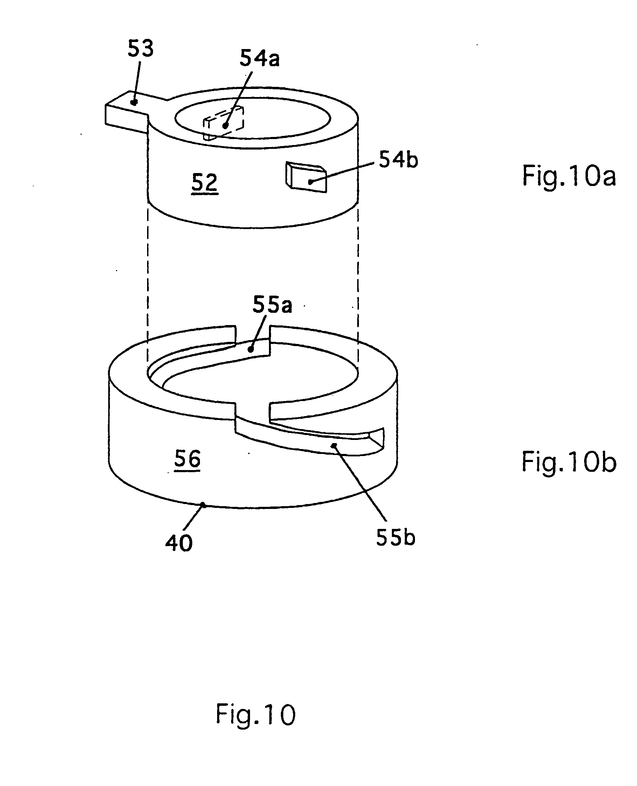

In Figur 10 ist eine andere Ausführung eines Schraub-Schub-Getriebes zum Einstellen eines Anschlages des Sprungstücks dargestellt. In diesem Beispiel hat der drehbare Stellring (52) auf seiner Außenwand zwei Nocken (54a; 54b). Der axial verschiebbare Ring (56) hat zwei Kulissen (55a; 55b), die Teile einer zweigängigen Schraubenfläche sind. Der Außendurchmesser des drehbaren Stellrings (52) ist fast so groß wie der Innendurchmesser des axial verschiebbaren Rings (56). Beim Ineinanderstecken der beiden Ringe greifen die beiden Nocken (54a; 54b) in die Kulissen (55a; 55b) ein. Beim Drehen des drehbaren Stellrings (52) gegen den axial verschiebbaren Ring (56) gleiten die Nocken in den Kulissen. Die Nocken verschieben den Ring (56) in axialer Richtung bei jeder Drehrichtung des drehbaren Stellrings. Eine Rückstellfeder für den axial verschiebbaren Ring ist bei dieser Ausführung nicht erforderlich.
Die Nocken beziehungsweise die Kulissen können den beiden Ringen in anderer Weise zugeordnet werden. Beispielsweise können die Nocken auf der Innenwand des axial verschiebbaren Ringes und die Kulissen in der Wand des drehbaren Stellrings angebracht sein. Es kann zweckmäßig sein, die radiale Tiefe der Kulissen kleiner zu wählen als die radiale Dicke des mit Kulissen versehenen Rings.
Die in Figur 10a dargestellten Nocken haben eine schraubenflächenartige Form. Ihre Höhe in axialer Richtung stimmt im Wesentlichen überein mit der Höhe der Kulissen in axialer Richtung. An Stelle solcher Nocken sind beispielsweise zylinderförmige Nocken geeignet, deren Durchmesser im Wesentlichen so groß ist wie die Höhe der Kulissen in axialer Richtung.

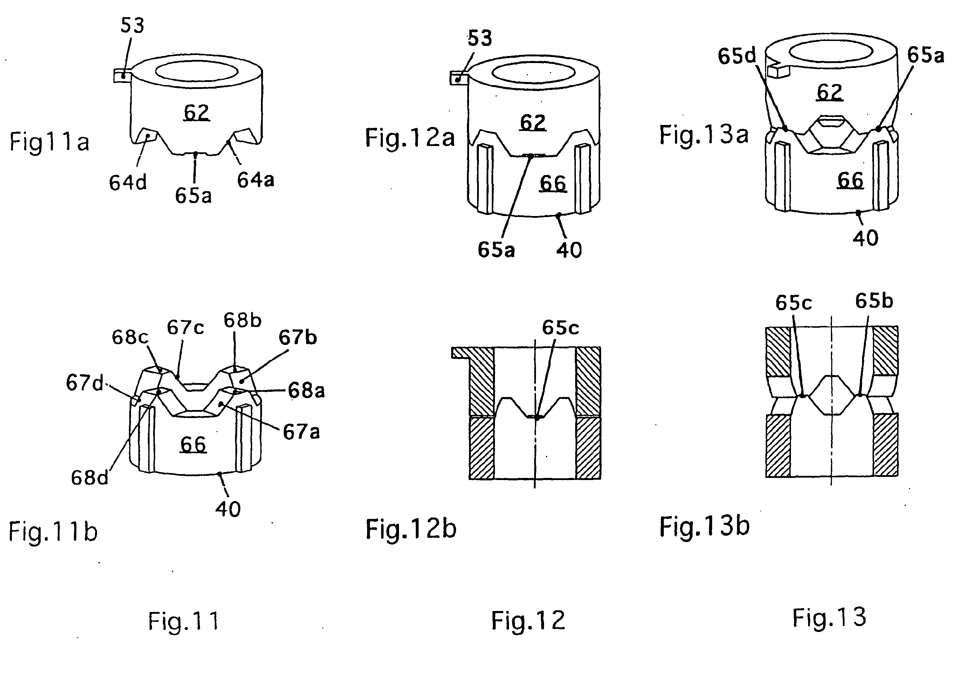

In Figur 11 ist eine weitere Ausführungsform eines drehbaren Stellrings (62) und eines axial verschiebbaren Rings (66) in Schrägansicht dargestellt. Beide Ringe haben eine viergängige Schraubenfläche, die mit jeweils zwei Raststufen versehen sind. Die Schraubenflächen (64a; 64d) am drehbaren Stellring sind in Figur 11a sichtbar; die beiden weiteren Schraubenflächen am drehbaren Stellring sind in Figur 11a verdeckt. Der axial verschiebbare Ring (66) hat die vier entsprechenden Schraubenflächen (67a; 67b; 67c; 67d), die mit den zugeordneten Schraubenflächen am drehbaren Stellring zusammenwirken.
Jede der vier Schraubenflächen an jedem der beiden Ringe geht in zwei Raststufen über, von denen jeweils die vier "hohen" Stufen in einer Ebene liegen und die vier "niedrigen" Stufen in einer dazu parallelen Ebene. Beide Ebenen stehen senkrecht auf der Achse des drehbaren Stellrings. Die "hohen" Stufen am drehbaren Stellring erstrecken sich jeweils über einen Winkel, der im Wesentlichen genau so groß ist wie jeweils der Winkel, über den sich die "niedrigen" Stufen am axial verschiebbaren Ring erstrecken. Die "niedrigen" Stufen am drehbaren Stellring erstrecken sich jeweils über einen Winkel, über den sich die "hohen" Stufen am axial verschiebbaren Ring erstrecken.
Die "hohen" Stufen am drehbaren Stellring können jeweils mit einer Aussparung versehen sein, von denen nur die Aussparung (65a) in einer dieser vier Stufen in Figur 11 a sichtbar ist; die Aussparungen in den anderen dieser "hohen" Stufen des drehbaren Stellrings sind in Figur 11a verdeckt. Die Aussparungen in den "hohen" Stufen des drehbaren Stellrings erstrecken sich jeweils über einen Winkel, der im Wesentlichen genau so groß ist wie der Winkel, über den sich die "hohen" Stufen des axial verschiebbaren Ringes erstrecken.
In Figur 12 sind die beiden Ringe des Schraub-Schub-Getriebes zum Einstellen eines Anschlages des Sprungstücks des Sperrspannwerkes in der Position dargestellt, in der die "hohen" Stufen des einen Rings in den "niedrigen" Stufen des anderen Rings liegen. Figur 12a zeigt diese Anordnung in Schrägansicht und Figur 12b im Längsschnitt durch die Achse der Ringe. In Figur 12b ist die nun sichtbare Aussparung (65c) enthalten.
In Figur 13 sind die beiden Ringe des Schraub-Schub-Getriebes zum Einstellen eines Anschlages des Sprungstücks des Sperrspannwerks in der Position dargestellt, in der die "hohen" Stufen beider Ringe übereinander liegen. Figur 13a zeigt die Anordnung in Schrägansicht und Figur 13b im Längsschnitt durch die Achse der beiden Ringe. In Figur 13a sind die beiden Aussparungen (65a; 65d) enthalten, in Figur 13b sind die nun sichtbaren Aussparungen (65b; 65c) enthalten.

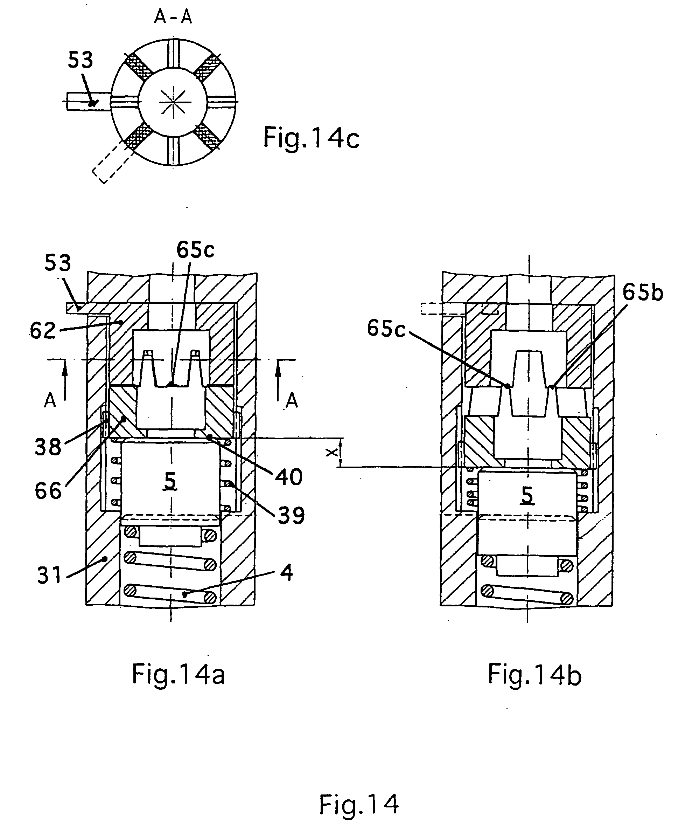

Figur 14 zeigt die Anordnung des Schraub-Schub-Getriebes zum Einstellen eines Anschlags des Sprungstücks innerhalb des Gehäuses im Längsschnitt durch die Achse des Gehäuses, die mit den Achsen der beiden Ringe zusammenfällt. Figur 14a zeigt die Position der beiden Ringe entsprechend Figur 12 und Figur 14b entsprechend Figur 13. In den Figuren 14a und 14b ist die Ganghöhe der Schraubenflächen überhöht dargestellt.

Das Gehäuse (31) enthält die Arbeitsfeder (4), deren eines Ende am Sprungstück (5) anliegt. Das Sprungstück liegt am Anschlag (40) an der einen Seite des axial verschiebbaren Ringes (66) an. Die viergängige Schraubenfläche des axial verschiebbaren Rings wirkt mit der Schraubenfläche des drehbaren Rings (62) zusammen. Der axial verschiebbare Ring (66) ist mittels der Führungsstege (38), die in Führungsnuten in der Wand des Gehäuses gleiten, gegen Drehen gesichert. Die Rückstellfeder (39) hält den axial verschiebbaren Ring in Kontakt zum drehbaren Stellring.
Figur 14c zeigt einen Querschnitt durch die Anordnung nach Figur 14a in Höhe der Ebene A - A.
In der erfindungsgemäßen Ausführung nach den Figuren 11 bis 14 kann der drehbare Stellring um 90 Grad zwischen den beiden verrasteten Positionen gedreht werden. Dabei verschiebt sich der axial verschiebbare Ring (66) und damit der eine Anschlag des Sprungstücks (5) um den Weg (X), der zwischen den beiden Figuren dargestellt ist.

Innerhalb des Rahmens der Erfindung gibt es weitere Möglichkeiten, den Weg des Sprungstücks zu verändern. Es kann zweckmäßig sein, den Weg des Sprungstücks in mehrere Teile aufzuteilen, die nacheinander durchfahren werden. Damit kann z.B. bei einem nadellosen Injektor die Flüssigkeitsmenge, die dem vom Schraub-Schub-Getriebe zum Spannen der Arbeitsfeder vorgegebenen Weg des Sprungstücks beim Übergang von der zweiten Ruhelage in die erste Ruhelage entspricht, in mehreren Teilmengen abgegeben werden, ohne das Sperrspannwerk des nadellosen Injektors zwischendurch erneut zu betätigen.

## Patentansprüche

1. Sperrspannwerk für einen miniaturisierten Hochdruckerzeuger mit federbetätigtem Abtrieb und mit einem veränderbaren Volumen der abzugebenden Flüssigkeit umfassend
• zwei Gehäuseteile (1; 2), die gegeneinander drehbar gelagert sind,
• eine Arbeitsfeder (4) als Speicher für die mechanische Energie,
• ein Sprungstück auf das die Arbeitsfeder (4) wirkt,
• eine Schraub-Schub-Getriebe, durch das die Arbeitsfeder (4) beim Drehen der beiden Gehäuseteile gespannt wird,
• einen ersten (8) Anschlag für das Sprungstück, der die Wegbegrenzung des Sprungstücks in dessen ersten Ruhelage definiert, wenn die Arbeitsfeder (4) gespannt ist,
• einen zweiten Anschlag (9), der die Wegbegrenzung für Sprungstück in dessen zweiter Ruhelage definiert, wenn die Arbeitsfeder (4) entspannt ist,
• einen mit dem Sprungstück verbundener Kolben (22; 26),
• einen Zylinder (21), in dem der Kolben mit der Bewegung des Sprungstücks in axialer Richtung bewegt wird,
• eine Düse (23) am Ende des Zylinders, durch die das Volumen der Flüssigkeit mittels des Kolbens ausgestoßen wird, und
• mindestens ein weiteres Bauteil, mit dem die Position eines der beiden Anschläge des Sprungstücks und damit der vom Sprungstück zuruckgelegte Weg veränderbar ist.

2. Sperrspannwerk nach Anspruch 1, wobei
• mindestens ein weiteres Bauteil vorgesehen ist, mit dem die Position des zweiten Anschlags (9) des Sprungstücks veränderbar ist.

3. Sperrspannwerk nach Anspruch 1, wobei
• der Kolben ein Hohlkolben (22, 26) ist.

4. Sperrspannwerk nach Anspruch 1, wobei
• das Sprungstück scheibenförmig oder tassenförmig ist.

5. Sperrspannwerk nach Anspruch 1, wobei
• das Sprungstück tassenförmig ist und auf dem Boden des tassenförmigen Sprungstücks (5) ein flacher Ring (10) als das mindestens eine weitere Bauteil liegt.

6. Sperrspannwerk nach Anspruch 1, wobei
• auf dem Boden des tassenförmigen Sprungstücks (5) eine zweiteilige Stufenscheibe (11a; 11b) als das mindestens eine weitere Bauteil liegt.

7. Sperrspannwerk nach Anspruch 1, wobei
• in dem oberen Gehäuseteil (1) mehrere Stellschrauben (12) - bevorzugt drei Stellschrauben - zur Bestimmung des zweiten Anschlags (9) als das mindestens eine weitere Bauteil vorgesehen sind.

8. Sperrspannwerk nach Anspruch 1, wobei
• das Sprungstück (5) mit einer Stellmutter (13) zur Bestimmung des zweiten Anschlags (9) als dem mindestens einen weiteren Bauteil versehen ist.

9. Sperrspannwerk nach Anspruch 1, wobei
• der Boden des tassenförmigen Sprungstücks (5) mit einem oder mehreren Löchern versehen ist, in die Stifte oder Schrauben (25) als das mindestens eine weitere Bauteil eingesetzt sind, die in das tassenförmige Sprungstück hineinragen, und die - gegebenenfalls mittels einer Platte (24) - den zweiten Anschlag (9) des Sprungstücks bestimmen.

10. Sperrspannwerk nach Anspruch 1, wobei
• der mit dem Sprungstück verbundene Kolben in einer zentrischen Schraube (27) als dem mindestens einen weiteren Bauteil befestigt ist, und das innere Ende der in das tassenförmige Sprungstück hineinragenden zentrischen Schraube (27) den zweiten Anschlag (9) des Sprungstücks bestimmt.

11. Sperrspannwerk nach Anspruch 1, wobei
• ein weiteres Schraub-Schub-Getriebe zum Einstellen eines Anschlages des Sprungstücks als das mindestens eine weitere Bauteil vorhanden ist.

12. Sperrspannwerk nach Anspruch 11, wobei
• das weitere Schraub-Schub-Getriebe zum Einstellen eines Anschlages des Sprungstücks mindestens einen drehbaren Stellring (32; 52) umfaßt, der einen aus dem Gehäuse herausragenden Griff (33) hat, und wobei
• der drehbare Stellring eine Schraubenfläche (34a; 34b) hat, die mit einer ihr in axialer Richtung gegenüber liegenden Schraubenfläche zusammenwirkt, und die gegenüber liegende Schraubenfläche an einem innerhalb des Gehäuses befindlichen axial verschiebbaren Bauteil angebracht ist.

13. Sperrspannwerk nach Anspruch 12, wobei
• die Schraubenfläche,. die der Schraubenfläche am drehbaren Stellring gegenüber liegt, an einem axial verschiebbaren Ring (36; 56) angebracht ist.

14. Sperrspannwerk nach Anspruch 12, wobei
• die Schraubenfläche, die der Schraubenfläche am drehbaren Stellring gegenüber liegt, am axial verschiebbaren Sprungstück (5) angebracht ist.

15. Sperrspannwerk nach Anspruch 12, wobei
• die Schraubenflächen am drehbaren Stellring und am axial verschiebbaren Bauteil einem eingängigen Gewinde entsprechen.

16. Sperrspannwerk nach Anspruch 12, wobei
• die Schraubenflächen am drehbaren Stellring und am axial verschiebbaren Bauteil einem mehrgängigen - bevorzugt einem zweigängigen (34a; 34b; 37a; 37b) oder einem dreigängigen - Gewinde entsprechen.

17. Sperrspannwerk nach Anspruch 12, wobei
• die Schraubenflächen am drehbaren Stellring (34a; 34b) beziehungsweise am axial verschiebbaren Ring (37a; 37b) jeweils auf den einander gegenüber liegenden Kanten jedes Ringes angebracht sind.

18. Sperrspannwerk nach Anspruch 12, wobei
• die eine Schraubenfläche als Kulisse (55a; 55b) in einer Wand des axial verschiebbaren Bauteils oder in einer Wand des drehbaren Stellringes angebracht ist, und die gegenüber liegende Wand des drehbaren Stellrings beziehungsweise die gegenüber liegende Wand des axial verschiebbaren Bauteils mit Nocken (54a; 54b) versehen ist, die in die Kulisse eingreifen und in ihr gleiten.

19. Sperrspannwerk nach einem der vorstehenden Ansprüche, wobei
• die Schraubenflächen abschnittsweise (41a; 41b; 42a; 42b) vorhanden sind.

20. Sperrspannwerk nach einem der vorstehenden Ansprüche, wobei
• der drehbare Stellring mit einer Rastung (35) versehen ist, die mit einer am Gehäuse vorhandenen Rastung zusammenwirkt.

21. Sperrspannwerk nach einem der vorstehenden Ansprüche, wobei
• beide Schraubenflächen mit jeweils einer Rastung versehen sind, bevorzugt mit Rastzähnen oder Raststufen, und beide Rastungen miteinander zusammenwirken.

22. Sperrspannwerk nach einem der vorstehenden Ansprüche, wobei
• die Arbeitsfeder (4), das Sprungstück, der erste Anschlag (8) und der zweite Anschlag (9) in dieser Reihenfolge axial übereinander angeordnet sind.

23. Verwendung des Sperrspannwerks für einen miniaturisierten Hochdruckerzeuger nach Anspruch 1 in einem Hochdruckzerstäuber, mit dem eine Flüssigkeit zerstäubt wird.

## Claims

1. Locking tensioning mechanism for a miniaturised high pressure generator with a spring-operated power takeoff and with a variable volume of the liquid to be delivered, comprising
● two housing parts (1; 2) mounted to be rotatable relative to one another,
● an operating spring (4) which acts as a store for the mechanical energy,
● a spring component on which the operating spring (4) acts,
● a helical thrust gear by which the operating spring (4) is tensioned as the two housing parts are rotated,
● a first (8) stop for the spring component which defines the travel limits of the spring component in its first resting position when the operating spring (4) is under tension,
● a second stop (9) which defines the travel limits of the spring component in its second resting position when the operating spring (4) is relaxed,
● a piston (22; 26) connected to the spring component,
● a cylinder (21) in which the piston is moved as the spring component moves in the axial direction,
● a nozzle (23) at the end of the cylinder, through which the volume of liquid is expelled by the piston, and
● at least one additional component with which the position of one of the two stops of the spring component, and hence the distance travelled by the spring component, can be altered.

2. Locking tensioning mechanism according to claim 1, wherein
● at least one additional component is provided with which the position of the second stop (9) of the spring component can be altered.

3. Locking tensioning mechanism according to claim 1, wherein
● the piston is a hollow piston (22, 26).

4. Locking tensioning mechanism according to claim 1, wherein
● the spring component is disc-shaped or cup-shaped.

5. Locking tensioning mechanism according to claim 1, wherein
● the spring component is cup-shaped and a flat ring (10) is disposed on the bottom of the cup-shaped spring component (5) as the minimum of one further component.

6. Locking tensioning mechanism according to claim 1, wherein
● a two-part stepped disc (11 a; 11b) is disposed on the bottom of the cup-shaped spring component (5) as the minimum of one additional component.

7. Locking tensioning mechanism according to claim 1, wherein
● in the upper housing part (1) are provided a plurality of adjustment screws (12) - preferably three adjustment screws - as the minimum of one additional component which determines the second stop (9).

8. Locking tensioning mechanism according to claim 1, wherein
● the spring component (5) is provided with a locknut (13) as the minimum of one additional component which determines the second stop (9).

9. Locking tensioning mechanism according to claim 1, wherein
● the bottom of the cup-shaped spring component (5) is provided with one or more holes into which pins or screws (25) are inserted as the minimum of one additional component, these pins or screws (25) projecting into the cup-shaped spring component and determining, optionally by means of a plate (24), the second stop (9) of the spring component.

10. Locking tensioning mechanism according to claim 1, wherein
● the piston connected to the spring component is secured in a central screw (27) as the minimum of one additional component, and the inner end of the central screw (27) projecting into the cup-shaped spring component determines the second stop (9) of the spring component.

11. Locking tensioning mechanism according to claim 1, wherein
● a further helical thrust gear is provided as the minimum of one additional component for setting a stop of the spring component.

12. Locking tensioning mechanism according to claim 11, wherein
● the further helical thrust gear for setting a stop of the spring component comprises at least one rotatable adjusting ring (32; 52) which has a handle (33) protruding from the housing, and wherein
● the rotatable adjusting ring has a helicoidal surface (34a; 34b) which cooperates with a helicoidal surface located opposite it in the axial direction, and the opposing helicoidal surface is provided on an axially movable component located within the housing.

13. Locking tensioning mechanism according to claim 12, wherein
● the helicoidal surface located opposite the helicoidal surface on the rotatable adjusting ring is provided on an axially movable ring (36; 56).

14. Locking tensioning mechanism according to claim 12, wherein
● the helicoidal surface located opposite the helicoidal surface on the rotatable adjusting ring is provided on the axially movable spring component (5).

15. Locking tensioning mechanism according to claim 12, wherein
● the helicoidal surfaces on the rotatable adjusting ring and on the axially movable component correspond to a single-threaded screw thread.

16. Locking tensioning mechanism according to claim 12, wherein
● the helicoidal surfaces on the rotatable adjusting ring and on the axially movable component correspond to a multiple-threaded - preferably a double-threaded (34a; 34b; 37a; 37b) or triple-threaded - screw thread.

17. Locking tensioning mechanism according to claim 12, wherein
● the helicoidal surfaces on the rotatable adjusting ring (34a; 34b) or on the axially movable ring (37a; 37b) are provided in each case on the opposite edges of each ring.

18. Locking tensioning mechanism according to claim 12, wherein
● one helicoidal surface is provided as a detent (55a; 55b) in a wall of the axially movable component or in a wall of the rotatable adjusting ring, and the opposite wall of the rotatable adjusting ring or the opposite wall of the axially movable component is provided with cams (54a; 54b) which engage in the detent and slide therein.

19. Locking tensioning mechanism according to one of the preceding claims, wherein
● the helicoidal surfaces are provided in parts (41a; 41b; 42a; 42b).

20. Locking tensioning mechanism according to one of the preceding claims, wherein
● the rotatable adjusting ring is provided with engaging means (35) which cooperate with engaging means provided on the housing.

21. Locking tensioning mechanism according to one of the preceding claims, wherein
● the two helicoidal surfaces are in each case provided with engaging means, preferably with engaging teeth or engaging steps, and the two engaging means cooperate with one another.

22. Locking tensioning mechanism according to one of the preceding claims, wherein
● the operating spring (4), the spring component, the first stop (8) and the second stop (9) are arranged axially above one another in this order.

23. Use of the locking tensioning mechanism for a miniaturised high pressure generator according to claim 1 in a high pressure generator which is used to atomise a liquid.

## Revendications

1. Mécanisme tendeur à blocage pour un générateur de haute pression miniaturisé avec une évacuation actionnée par ressort et avec un volume du liquide à délivrer modifiable comprenant
● deux parties de boîtier (1 ; 2) qui sont montées de manière à pouvoir tourner l'une par rapport à l'autre,
● un ressort de travail (4) comme accumulateur pour l'énergie mécanique,
● un organe de détente sur lequel agit le ressort de travail (4),
● un organe de poussée à vis par lequel le ressort de travail (4) est tendu lors de la rotation des deux parties de boîtier,
● une première (8) butée pour l'organe de détente qui définit la limite de course de l'organe de détente dans sa première position de repos quand le ressort de travail (4) est tendu,
● une seconde butée (9) qui définit la limite de course de l'organe de détente dans sa deuxième position de repos quand le ressort de travail (4) est détendu,
● un piston (22 ; 26) relié à l'organe de détente,
● un cylindre (21) dans lequel le piston est déplacé en direction axiale avec le déplacement de l'organe de détente,
● une buse (23) à l'extrémité du cylindre par laquelle le volume de liquide est expulsé au moyen du piston, et
● au moins un autre élément de construction avec lequel la position de l'une des deux butées de l'organe de détente et donc la course parcourue par l'organe de détente sont modifiables.

2. Mécanisme tendeur à blocage selon la revendication 1 où
● il est prévu au moins un autre élément de construction avec lequel la position de la deuxième butée (9) de l'organe de détente est modifiable.

3. Mécanisme tendeur à blocage selon la revendication 1 où
● le piston est un piston creux (22, 26).

4. Mécanisme tendeur à blocage selon la revendication 1 où
● l'organe de détente est en forme de disque ou en forme de tasse.

5. Mécanisme tendeur à blocage selon la revendication 1 où
● l'organe de détente est en forme de tasse et un anneau plat (10) est situé sur le fond de l'organe de détente (5) en forme de tasse à titre du au moins un autre élément de construction.

6. Mécanisme tendeur à blocage selon la revendication 1 où
● un disque étagé en deux parties (11a ; 11b) est situé sur le fond de l'organe de détente (5) en forme de tasse à titre du au moins un autre élément de construction.

7. Mécanisme tendeur à blocage selon la revendication 1 où
● il est prévu dans la partie supérieure de boîtier (1) plusieurs vis de réglage (12) - de préférence trois vis de réglage - pour la définition de la deuxième butée (9) à titre du au moins un autre élément de construction.

8. Mécanisme tendeur à blocage selon la revendication 1 où
● l'organe de détente (5) est muni d'un écrou de réglage (13) pour la définition de la deuxième butée (9) à titre du au moins un autre élément de construction.

9. Mécanisme tendeur à blocage selon la revendication 1 où
● le fond de l'organe de détente (5) en forme de tasse est muni d'un ou plusieurs trous dans lesquels des tiges ou vis (25) font saillie à titre du au moins un autre élément de construction, qui pénètrent dans l'organe de détente en forme de tasse et qui déterminent la deuxième butée (9) de l'organe de détente - éventuellement au moyen d'une plaque (24).

10. Mécanisme tendeur à blocage selon la revendication 1 où
● le piston relié à l'organe de détente est fixé dans une vis centrale (27) à titre du au moins un autre élément de construction, et l'extrémité interne de la vis centrée (27) faisant saillie dans l'organe de détente en forme de tasse détermine la deuxième butée (9) de l'organe de détente.

11. Mécanisme tendeur à blocage selon la revendication 1 où
● un autre organe de poussée à vis pour le réglage d'une butée de l'organe de détente est présent à titre du au moins un autre élément de construction.

12. Mécanisme tendeur à blocage selon la revendication 11 où
● l'autre organe de poussée à vis pour le réglage d'une butée de l'organe de détente comprend au moins un anneau de réglage rotatif (32 ; 52) qui a une poignée (33) qui fait saillie du boîtier, et où
● l'anneau de réglage rotatif a une surface hélicoïdale (34a ; 34b) qui coopère avec une surface hélicoïdale qui lui est opposée en direction axiale, et la surface hélicoïdale opposée est disposée sur un élément de construction déplaçable axialement situé à l'intérieur du boîtier.

13. Mécanisme tendeur à blocage selon la revendication 12 où
● la surface hélicoïdale qui est opposée à la surface hélicoïdale sur l'anneau de réglage rotatif est disposée sur un anneau (36 ; 56) déplaçable axialement.

14. Mécanisme tendeur à blocage selon la revendication 12 où
● la surface hélicoïdale qui est opposée à la surface hélicoïdale sur l'anneau de réglage rotatif est disposée sur l'organe de détente (5) déplaçable axialement.

15. Mécanisme tendeur à blocage selon la revendication 12 où
● les surfaces hélicoïdales sur l'anneau de réglage rotatif et sur l'élément de construction déplaçable axialement correspondent à un filetage à un filet.

16. Mécanisme tendeur à blocage selon la revendication 12 où
● les surfaces hélicoïdales sur l'anneau de réglage rotatif et sur l'élément de construction déplaçable axialement correspondent à un filetage à plusieurs filets - de préférence à un filetage à deux filets (34a ; 34b ; 37a ; 37) ou à un filetage à trois filets.

17. Mécanisme tendeur à blocage selon la revendication 12 où
● les surfaces hélicoïdales sur l'anneau de réglage rotatif (34a ; 34b) respectivement sur l'anneau (37a ; 37b) déplaçable axialement sont formées chacune sur les bords opposés de chaque anneau.

18. Mécanisme tendeur à blocage selon la revendication 12 où
● une surface hélicoïdale est formée sous forme de coulisse (55a ; 55b) dans une paroi de l'élément de construction déplaçable axialement ou dans une paroi de l'anneau de réglage rotatif, et la paroi opposée de l'anneau de réglage rotatif respectivement la paroi opposée de l'élément de construction déplaçable axialement est munie d'ergots (54a ; 54b) qui pénètrent dans la coulisse et qui glissent dans celle-ci.

19. Mécanisme tendeur à blocage selon l'une des revendications précédentes où
● les surfaces hélicoïdales sont présentes par segments (41a ; 41b ; 42a; 42b).

20. Mécanisme tendeur à blocage selon l'une des revendications précédentes où
● l'anneau de réglage rotatif est muni d'une série d'encoches (35) qui coopère avec une série d'encoches présente sur le boîtier.

21. Mécanisme tendeur à blocage selon l'une des revendications précédentes où
● les deux surfaces hélicoïdales sont munies chacune d'une série d'encoches, de préférence de dents à encoches ou de gradins à encoches, et les deux séries d'encoches coopèrent entre elles.

22. Mécanisme tendeur à blocage selon l'une des revendications précédentes où
● le ressort de travail (4), l'organe de détente, la première butée (8) et la deuxième butée (9) sont disposés axialement les uns au dessus des autres dans cet ordre.

23. Utilisation du mécanisme tendeur à blocage pour un générateur de haute pression miniaturisé selon la revendication 1 dans un pulvérisateur haute pression avec lequel un liquide est pulvérisé.
